# EUROPÄISCHE PATENTSCHRIFT

(11) **EP 3 866 721 B1**
(45) Veröffentlichungstag und Bekanntmachung des Hinweises auf die Patenterteilung: **27.04.2022**
(21) Anmeldenummer: 19790174.7
(22) Anmeldetag: 15.10.2019
(51) Int. Cl.: A61B 50/20, A61B 17/86, A61B 50/22, A61B 50/30, A61B 50/33, A61B 17/00

(54) **RESORBIERBARE VERPACKUNG FÜR EIN MEDIZINTECHNISCHES PRODUKT**
RESORBABLE PACKAGING FOR A MEDICAL PRODUCT
EMBALLAGE RÉSORBABLE POUR PRODUIT MÉDICO-TECHNIQUE

(30) Priorität: 16.10.2018 DE 102018125610
(43) Veröffentlichungstag der Anmeldung: 25.08.2021
(73) Patentinhaber: Aesculap AG, 78532 Tuttlingen (DE)
(72) Erfinder: LENZENHUBER, Frederick, 78532 Tuttlingen (DE)
(74) Vertreter: Winter, Brandl - Partnerschaft mbB
(86) Internationale Anmeldenummer: PCT/EP2019/078013
(87) Internationale Veröffentlichungsnummer: WO 2020/079028

(56) Entgegenhaltungen:
- WO-A2-2005/118066
- DE-A1-102013 004 168

## Beschreibung

Die vorliegende Erfindung betrifft eine Verpackung, insbesondere eine Sterilverpackung, für ein medizintechnisches Produkt, mit einem Aufnahmeraum für das medizintechnische Produkt. Sie betrifft außerdem eine Verpackungseinheit mit einem solchen Produkt. Eine derartige Verpackung ist aus Dokument WO2005/118066A2 bekannt.

Zur Verpackung von medizintechnischen Produkten sind verschiedene Verpackungen und Vorrichtungen bekannt. Verpackungen von medizintechnischen Produkten wie Implantaten und Werkzeugen mit spitzen und/oder scharfen Wirkabschnitten, beispielsweise in Form von Schneid- oder Sägekanten, Fräsköpfen und Bohrern oder Gewindeabschnitten, unterliegen besonderen Anforderungen. Denn neben dem Schutz des verpackten Produkts vor ungewünschten Einflüssen wie Verschmutzung und Beschädigung ist außerdem ein Schutz von Anwendern vor Verletzungen vor und insbesondere nach einer Verwendung des Produkts sicherzustellen.

Im Hinblick auf den Schutz des Produkts ist anzumerken, dass bei in einer Umverpackung verpackten medizintechnischen Produkten ein grundsätzliches Risiko besteht, dass es zu einem ggf. versehentlichen Kontakt zwischen dem Produkt und Teilen der Verpackung kommt. Insbesondere bei Kontakt von scharfen Wirkabschnitten mit dem Verpackungsmaterial kann es zu Abrasion kommen, so dass Teile und/oder Partikel des Verpackungsmaterials am Produkt anhaften und über das Produkt in einen Patienten verbracht werden können. Die vorstehend beschriebene Problematik ist besonders relevant bei Produkten wie Implantaten oder chirurgischen Werkzeugen. Derartige Produkte besitzen in der Regel scharfe Körperkonturen oder Schneidkanten, die bei Kontakt insbesondere schon während einer Handhabung (Entnahme des Produkts aus der Verpackung) mit der Umverpackung von dieser Material abtragen können, zum Beispiel in Form winziger Partikel. Solche Materialbestandteile haften dann in der Regel nicht oder nur schlecht wahrnehmbar am Produkt an und können in nachteiliger Weise im Rahmen einer bestimmungsgemäßen Verwendung des Produkts, wie im Rahmen eines Einsatzes während einer OP, in den Körper eines Patienten eingebracht werden. Möglicherweise verbleiben diese Materialpartikel im Patenten und können dort schlimmstenfalls Entzündungsreaktionen, allergische Reaktionen und dergleichen hervorrufen.

Aus dem Stand der Technik sind Verpackungen bekannt, in denen das medizintechnische Produkt fest und/oder lagedefiniert gehalten ist, die aber die vorstehend beschriebene Problematik nicht zufriedenstellend lösen können. Ein Beispiel für solche Verpackungen ist eine Blisterverpackung mit einer Klemmung im Kupplungsbereich eines Produkts in Form eines Werkzeugs, um dieses in einer definierten Lage in der Verpackung zu positionieren und zu halten. Ein Nachteil ist, dass, falls es durch äußere Einwirkung auf die Blisterverpackung verbunden mit deren relativ geringer Stabilität zu einem Lösen der Klemmung des Werkzeugs darin kommt, das Werkzeug in der Verpackung verrutschen und insbesondere mit seinen scharfen Wirkkanten an die Verpackung stoßen kann. Wie bereits in der DE10 2013 004 168 A1 dargelegt wurde, können dadurch in unerwünschter Weise mikrofeine Partikel der Verpackung in den Bereich der Schneiden des Werkzeuges gelangen und von dort gegebenenfalls in eine Operationswunde eingetragen werden, wodurch es zu schweren Gewebeirritationen kommen kann. Infolgedessen können derartige Verpackungen die vorstehend beschriebene Problematik nur bedingt lösen, da es insbesondere bei der Handhabung des Produkt während der Entnahme aus der Umverpackung zu einer Kontaminierung durch Verpackungsmaterial kommen kann.

Zur Verbesserung dieser Umstände sieht die DE 10 2013 004168 A1 eine Verpackung von sterilen Operationswerkzeugen vor, bei der das Operationswerkzeug unter sterilen Bedingungen in einer Kunststoffverpackung verpackt ist, die Verpackung mindestens zweiteilig ausgebildet ist und aus einer inneren Schutzverpackung für empfindliche Teile des Operationswerkzeugs und einer die Schutzverpackung aufnehmenden, siegelfähigen Blisterverpackung besteht. Außerdem wird ein Verfahren zur Verpackung von sterilen Operationswerkzeugen vorgeschlagen, bei dem das Operationswerkzeug unter sterilen Bedingungen in der Kunststoffverpackung verpackt wird, wobei in einem ersten Schritt zunächst das Operationswerkzeug mindestens teilweise und insbesondere mit seinen empfindlichen Schneidkanten und Arbeitsflächen in der ersten Schutzverpackung verpackt wird und die Schutzverpackung zusammen mit dem dort verpackten Operationswerkzeug in eine zweite Blisterverpackung verbracht wird, dort lagengesichert festgelegt wird und danach mit einer Siegelfolie verschlossen wird. Die vorstehend beschriebene Problematik einer Kontaminierung des Produkts bei der Entnahme aus der Verpackung besteht aber auch dabei.

Vor diesem Hintergrund liegt der vorliegenden Erfindung die Aufgabe zugrunde, die genannten Nachteile des Stands der Technik zu verringern, insbesondere eine Verpackung für ein medizintechnisches Produkt zur Verfügung zu stellen, mit der das Risiko von negativen Folgen, wie Entzündungen, allergischen Reaktionen und dergleichen, für einen Patienten durch verschleppten Abrieb minimiert werden kann.

Diese Aufgabe wird nach der vorliegenden Erfindung gelöst durch eine Verpackung nach Anspruch 1, also eine Verpackung, insbesondere Sterilverpackung, für ein medizintechnisches Produkt, mit einem Aufnahmeraum für das medizintechnische Produkt, wobei ein dem Aufnahmeraum zugewandter Abschnitt der Verpackung zumindest teilweise, vorzugsweise vollständig, aus einem resorbierbaren Material besteht, einer Umverpackung und einer in dem Aufnahmeraum angeordneten Haltevorrichtung, insbesondere einer lagebestimmt in dem Aufnahmeraum angeordneten Haltevorrichtung, in der das Produkt gehalten, insbesondere lagebestimmt gehalten ist. Die vorstehende Aufgabe wird außerdem gelöst durch eine Verpackungseinheit mit einer erfindungsgemäßen Verpackung, insbesondere einer Verpackung nach einem der angehängten Ansprüche, und einem in dem Aufnahmeraum angeordneten medizintechnischen Produkt.

Bei dem Produkt kann es sich im Rahmen der Erfindung insbesondere um ein Produkt mit einem Arbeitsende im weitesten Sinne handeln. Ein solches Arbeitsende kann zum Beispiel ein distaler, in der Regel scharfkantiger Wirkabschnitt zum Erfüllen einer operativen Funktion eines Werkzeug / Operationswerkzeug sein, das des Weiteren einen proximalen Koppelabschnitt zum Koppeln des Werkzeugs mit einer Griffeinheit, insbesondere einer Antriebsgriffeinheit, aufweist. Beispiele für solche Werkzeuge sind vor allem rotierende Werkzeuge sowie Sägewerkzeuge, unter anderem Bohrer, Fräser, Sägen, Schraubaufsätze, Schneidklingen oder Schleifaufsätze, die in allgemein bekannter Weise mit einer Griff- und/oder Antriebseinheit gekoppelt werden. Weitere Beispiele für ein Produkt im Sinne der Erfindung sind atraumatisch wirkende Instrumente wie HF-Spritzen oder Ultraschallklingen. Außerdem kann es sich bei dem Produkt um ein Implantat beliebiger Art und Form handeln, wie zum Beispiel Knochen- und Gelenkimplantate oder -teilimplantate, Stants, etc. Solche Implantate besitzen oftmals einen scharfkantigen Wirkabschnitt, zum Beispiel in Form eines Gewindes oder ähnlichem.

Nach der Erfindung ist das Produkt in die zumindest teilweise aus einem resorbierbaren Material bestehende Verpackung einbringbar bzw. eingebracht. Erfindungsgemäß kann die gesamte Verpackung oder aber nur ein Teil davon aus dem resorbierbaren Material bestehen. Sollten nur Teilbereiche der Verpackung aus resorbierbarem Material bestehen, so sind diese Teilbereiche vorzugsweise derart angeordnet und ausgebildet, dass ein Kontakt des Produkts mit nicht aus resorbierbarem Material bestehenden Teilen der Verpackung beim Einbringen, Aufbewahren/Lagern und Entnehmen des Produkts sicher vermieden werden kann. So kann denn im einem Fall, wenn es zu Kontakt zwischen dem Produkt und der Verpackung kommt, und dabei am Produkt anhaftende Materialpartikel der Verpackung unbemerkt in den Körper eines Patienten verschleppt werden, sicher ein negativer Einfluss auf den Patienten vermieden oder zumindest verringert werden, da die am Produkt ggf. anhaftenden Materialpartikel in vorteilhafter Weise durch den Körper des Patienten abgebaut werden. Vorzugsweise besteht die Verpackung vollständig aus einem resorbierbaren Material. Unter einem resorbierbaren Material im Sinne der Erfindung ist insbesondere ein Material zu verstehen, dass durch den menschlichen oder einen tierischen Körper zersetzt und abgebaut und ggf. ausgeschieden werden kann, insbesondere in für den Körper unbedenkliche Bestandteile wie Wasser und Kohlendioxid.

Vorteilhafte Ausführungsformen der Erfindung sind in den Unteransprüchen beansprucht und werden nachfolgend näher erläutert.

Eine Ausführungsform der Erfindung ist dadurch gekennzeichnet, dass die Verpackung die Umverpackung aufweist. Zusätzlich weist sie eine in dem Aufnahmeraum angeordnete Haltevorrichtung auf. Eine solche Haltevorrichtung ist in vorteilhafter Weise lagebestimmt in dem Aufnahmeraum angeordnet und/oder gehalten. Sie dient insbesondere dazu, das Produkt zu halten, insbesondere lagebestimmt zu halten und im Aufnahmeraum derart zu positionieren, dass ein Kontakt von scharfen Wirkabschnitten mit dem Verpackungsmaterial vermieden wird und scharfe Wirkabschnitte von der Verpackung beabstandet sind. Durch Vorsehen einer solchen Haltevorrichtung kann ein Kontakt des Produkts mit Teilen der Verpackung verhindert oder zumindest sicher minimiert werden, so dass Abrieb von Verpackungsmaterial durch das Produkt verhindert werden kann. Vorzugsweise besteht bzw. bestehen die Haltevorrichtung und/oder die Umverpackung zumindest teilweise, vorzugsweise vollständig, aus einem resorbierbaren Material.

Nach einer weiteren Ausführungsform kann bzw. können die Haltevorrichtung und/oder die Umverpackung in ihrem den Aufnahmeraum begrenzenden Bereich mit einer Beschichtung aus dem resorbierbaren Material versehen sein. Dies hat den Vorteil, dass die Verpackung besonders einfach und günstig mit den jeweils gewünschten Eigenschaften, wie Gewicht, Stabilität, Größe, etc., hergestellt werden kann, wobei dennoch Belastungen des Patienten durch ein Verbringen von Verpackungsmaterial in dessen Körper sicher vermieden werden können. Die Dicke der Beschichtung ist vorzugsweise derart ausgebildet, dass im Falle eines Kontakts zwischen Produkt und Umverpackung und/oder Haltevorrichtung es zu keinem Abrieb von nicht resorbierbarem Material kommt. Ein besonderer Vorteil ist, dass bei dieser Ausführungsform der Erfindung die Umverpackung und/oder die Haltevorrichtung weitgehend unabhängig von den mechanischen Eigenschaften des resorbierbaren Materials designed werden können, so dass bestehende und bewährte Designs derartiger Verpackungen im Rahmen der Erfindung weitgehend übernommen werden können. In einer vorteilhaften Weiterbildung dieser Ausführungsform kann die Beschichtung dabei in Form einer Folierung auf die Haltevorrichtung und/oder die Umverpackung aufgebracht sein, d.h. es kann ein zweites sich von dem Material der Haltevorrichtung und/oder der Umverpackung unterscheidendes, resorbierbares Material, beispielsweise durch Verschweißen oder Verkleben, auf die Haltevorrichtung und/oder die Umverpackung aufgebracht werden. Anders ausgedrückt kann die Haltevorrichtung und/oder die Umverpackung mit diesem zweiten Material bzw. der Folie überzogen werden, so dass einfach die Schichtdicke des resorbierbaren Materials gesteuert werden kann und somit auch größere Schichtdicken realisiert werden können.

Bei dem resorbierbaren Material im Sinne der Erfindung kann es sich um ein resorbierbares Biopolymer oder ein Copolymer davon, insbesondere um einen resorbierbaren Polyester, Gelatine, thermoplastische Stärke (TPS) oder Zusammensetzungen oder Mischungen daraus handeln. Vorzugsweise wird als resorbierbares Material im Sinne der Erfindung ein Polyaktid (PLA), insbesondere ein Polyaktid der chemischen Formel (C3H4O2)n-O-CO-CH(CH4), insbesondere ein Poly-L-Laktid (PLLA), ein Poly-D-Laktid (PDLA) oder ein Poly-DL-Laktid (PDLLA), PCL (Polycaprolactan), PHB (Polyhydroxybuttersäure) oder eine Zusammensetzung oder eine Mischung aus den vorstehend genannten Materialen verwendet. Diese resorbierbaren Materialien sichern nicht nur einen kontrollierbaren und sicheren biologischen Abbau im Köper, sondern sind außerdem in höchstem Maß gewebeverträglich. Im Falle von PLA spaltet im Körper des Patienten vorhandenes Wasser die Polymerketten dieses Materials auf und der menschliche Stoffwechsel wandelt die D- und L-Laktide letztlich in Kohlendioxid und Wasser um, so dass versehentlich in den Körper des Patienten verbrachte Materialpartikel in besonders vorteilhafter Weise rückstandsfrei abgebaut werden. Die Erfindung ist besonders geeignet für medizintechnische Instrumente und Werkzeuge, die scharfkantige und empfindliche Wirk- oder Arbeitskonturen aufweisen, zum Beispiel scharfe Schneiden, Klingen, Bohr-, Säge- und Fräskonturen, die besonders leicht einen Abrieb und ein Anhaften von Verpackungsmaterial bewirken können.

Nach einer weiteren Ausführungsform der Erfindung ist vorgesehen, dass die Haltevorrichtung von der Umverpackung separat ausgebildet ist. Alternativ oder zusätzlich kann die Haltevorrichtung ausgebildet sein, das Produkt zumindest bereichsweise, vorzugsweise vollständig von der Umverpackung zu beabstanden. Wenn es sich bei dem Produkt um ein medizintechnisches Werkzeug handelt, kann dieses insbesondere im Bereich eines Wirkabschnitts des Werkzeugs von der Umverpackung beabstandet gehalten werden. Handelt es sich bei dem Produkt um ein Implantat mit scharfkantigen Wirkabschnitten oder Strukturen, wie zum Beispiel ein Gewinde, können solche Strukturen einfach von der Umverpackung beabstandet gehalten werden. Auf diese Weise kann ein Abrieb von Verpackungsmaterial durch das Produkt einfach limitiert oder sogar verhindert werden.

Nach einer bevorzugten Ausführungsform ist zumindest der Wirkabschnitt des Werkzeugs bzw. ggf. vorhandene scharfkantige Strukturen des Implantats in der Haltevorrichtung exponiert und hat weder unmittelbaren Kontakt zur Haltevorrichtung noch zur Umverpackung. Anders ausgedrückt ist die Haltevorrichtung zumindest im Bereich des Wirkabschnitts des Werkzeugs / scharfkantiger Strukturen eines Implantats offen. Dadurch wird unter anderem der Vorteil erzielt, dass der Wirkabschnitt / die scharfkantige Struktur für einen Nutzer nahezu uneingeschränkt sichtbar ist, was eine Identifikation des Werkzeugs / des Implantats erleichtert. Außerdem ist der Wirkabschnitt für Medien wie Desinfektions- und Sterilisationsmittel frei zugänglich. Dies bewirkt den großen Vorteil, dass die erfindungsgemäße Verpackung sowohl für sterilisierte als auch unsterilisierte Produkte geeignet ist. Letztgenannte können in besonders vorteilhafter Weise zusammen mit der Halteeinrichtung aus der Umverpackung genommen und sterilisiert werden.

Nach einer Ausführungsform der Erfindung ist die Haltevorrichtung separat von der Umverpackung ausgebildet. Daher kann das in der Haltevorrichtung gehaltene Produkt / Werkzeug / Implantat zusammen mit dieser aus der Umverpackung entnommen werden und insbesondere auch nach dem Auspacken bis zum letztlichen Gebrauch in der Haltevorrichtung verbleiben. Vorzugsweise sind Verpackung und Haltevorrichtung derart ausgebildet, dass ein Entnehmen durch Handhabung der Haltevorrichtung und ohne direkte Kontaktierung des Produkts / Werkzeugs / Implantats erfolgen kann, so dass die Wahrscheinlichkeit einer Kontaminierung des Produkts / Werkzeugs / Implantats einfach und sicher minimiert, wenn nicht sogar vermieden werden kann. Außerdem kann das Produkt / Werkzeug / Implantat zusammen mit der Haltevorrichtung auf einem Instrumentiertisch platziert werden, ohne es lose ablegen zu müssen. Während einer Operation oder Behandlung kann die Halterung in besonders vorteilhafter Weise zur Zwischenlagerung des Produkts / Werkzeugs / Implantats verwendet werden, was zu besserer Ordnung und Übersichtlichkeit führt.

Da zumindest der Wirkabschnitt, vorzugsweise das gesamte Produkt / Werkzeug / Implantat, von der Verpackung beabstandet ist, kann mit besonderem Vorteil eine kontaktfreie Lagerung im Bereich des Wirkabschnitts und darin ggf. vorhandenen Schneiden oder scharfen Kanten sicher gewährleistet werden. Insbesondere ist ein Kontakt des Wirkabschnitts zu allen umliegenden Verpackungskomponenten wie zum Beispiel Blister, Tyvekfolie, Träger, etc. nicht möglich. Eine unerwünschte gegenseitige Beeinflussung von Umverpackung und Produkt / Werkzeug / Implantat, wie zum Beispiel Materialabtrag oder Perforierung der Umverpackung oder Abstumpfen oder Verunreinigen kann sicher verhindert werden.

Insgesamt stellt die Erfindung damit neben der Verpackung im eigentlichen Sinne durch die separate Haltevorrichtung eine eigenständige Halterung zur Verfügung, die neben einer sicheren und stabilen Lagerung von Produkten / Werkzeugen / Implantaten während des Transports in der jeweiligen Umverpackung außerdem eine Möglichkeit zur definierten Positionierung und sicheren Handhabung des Werkzeugs im Rahmen einer Aufbereitung (z.B. Sterilisierung) sowie bei einer Bereitstellung des Produkts während einer Behandlung im OP ermöglicht. Dazu kann die Haltevorrichtung zusammen mit dem darin gehaltenen Produkt / Werkzeug / Implantat aus der Umverpackung genommen und aufgestellt werden.

Eine Ausführungsform der Erfindung ist dadurch gekennzeichnet, dass der Aufnahmeraum durch die Umverpackung geschlossenen ausgebildet ist, insbesondere hermetisch dicht. Er kann außerdem steril ausgebildet sein. Durch die Erfindung und die das Produkt / Werkzeug / Implantat von der Verpackung beabstandet haltende Haltevorrichtung kann die Sterilität und Unversehrtheit der Verpackung in einfacher und zuverlässiger Weise sichergestellt werden.

Nach einer Ausführungsform der Erfindung weist die Haltevorrichtung eine Grundplatte auf. Diese kann insbesondere derart ausgebildet sein, dass sie eine Standfläche zum lagestabilen Aufstellen der Haltevorrichtung mit einem darin gehaltenen Produkt, insbesondere in einem aus der Verpackung ausgepackten Zustand, und/oder eine Anlagestruktur zur Anlage an der Verpackung ausbildet. Neben der Standfläche kann die Haltevorrichtung weitere Anlagestrukturen oder -abschnitte aufweisen, die mit der Verpackung zusammenwirken und an dieser anliegen, so dass eine eindeutige und stabile Lagepositionierung in der Aufnahme der Verpackung gewährleistet ist.

Die Haltevorrichtung kann nach einer Ausführungsform der Erfindung zumindest zwei voneinander beabstandete Haltestrukturen zum lagebestimmten Positionieren des Produkts aufweisen. Bevorzugt sind diese jeweils an der Grundplatte angeordnet und können sich von dieser in den Aufnahmeraum hinein erstrecken. Neben der Funktion des Haltens des Produkts können die Haltestrukturen Anlagen ausbilden, mit denen die Haltevorrichtung an der Verpackung anliegt, vorzugsweise derart, dass sie in der Aufnahme der Verpackung lagegesichert oder lagefixiert ist.

Eine Ausführungsform ist dadurch gekennzeichnet, dass eine der Haltestrukturen endseitig an der Haltevorrichtung, insbesondere an der Grundplatte, angeordnet ist. Eine andere der Haltestrukturen kann im Wesentlichen mittig an der Haltevorrichtung, insbesondere an der Grundplatte, angeordnet sein. Dies ermöglicht eine besonders stabile Halterung des Produkts in der Haltevorrichtung.

Vorzugsweise ist eine der Haltestrukturen zum lagebestimmten Positionieren des Produkts relativ zur Haltevorrichtung in einer ersten Richtung ausgebildet. Die andere der Haltestrukturen kann zum lagebestimmten Positionieren des Produkts relativ zur Haltevorrichtung in einer zweiten Richtung und einer dritten Richtung ausgebildet sein, wobei die erste, zweite und dritte Richtung zueinander orthogonal sind. Durch eine derartige Führung ist das Produkt einerseits stabil in der Haltevorrichtung gehalten, andererseits kann es besonders einfach durch einen Anwender in der Haltevorrichtung angeordnet und aus der Haltevorrichtung entnommen werden.

Es ist besonders vorteilhaft, wenn nach einer weiteren Ausführungsform der Verpackung zumindest eine der Haltestrukturen mit dem Produkt einen Formschluss und/oder einen Kraftschluss ausbildet, insbesondere mit einer Koppelstruktur des Produkts.

Zumindest eine der Haltestrukturen, insbesondere beide Haltestrukturen, kann bzw. können jeweils zwei einander gegenüberliegende Haltearme aufweisen. Zwischen diesen ist jeweils ein Schlitz zur Aufnahme und Halterung des Produkts ausgebildet. Die Haltearme können im Wesentlichen senkrecht zur Grundplatte der Haltevorrichtung angeordnet sein. Sie können in besonders vorteilhafter Weise als Klemmarme ausgebildet sein und insbesondere in einer Richtung quer zum Schlitz federnde Eigenschaften aufweisen. Ein Entnehmen sowie ein Anordnen des Produkts in den Haltestrukturen ist besonders einfach möglich, wenn nach einer Form der Erfindung sich der Schlitz von proximal der Basis in Richtung distal der Basis aufweitet. Dies begünstigt eine weitgehend gefahrlose Handhabung sowohl von ungebrauchten als auch von gebrauchten Instrumenten, so dass insbesondere eine verletzungsfreie Entsorgung von Produkten in der Halterung verbessert und die dabei grundsätzlich bestehende Infektionsgefahr verringert wird.

Nach einer Fortbildung der Erfindung können die dem Schlitz zugewandten Seiten der Haltearme mit Einbuchtungen versehen sein. Solche Einbuchtung können in vorteilhafter Weise Rastbuchten für das in den Schlitz eingebrachte Produkt bilden, so dass dieses sicher und dennoch für einen Nutzer einfach entnehmbar in den Haltestrukturen gehalten ist.

Eine besonders nutzerfreundliche, da Verletzungen eines Nutzers an dem in der Haltevorrichtung vermeidende Ausführungsform der Erfindung sieht vor, dass die Haltevorrichtung eine Schutzlasche aufweist, die von der Grundplatte in den Aufnahmeraum hineinragt und ein distales Ende des Werkzeugs oder eine scharfkantige Struktur des Produkts berührungslos abdeckt. Auf diese Weise ist ein Anwender beim Handhaben der Haltevorrichtung mit dem darin gehaltenen Produkt vor Verletzungen durch gegebenenfalls scharfe Kanten des Produkts geschützt.

Nach einer Ausführungsform der Erfindung ist die Verpackung als Blister ausgebildet. Solche Blister sind günstig und können für nahezu beliebige Formen von Produkt und/oder Haltevorrichtung hergestellt werden. Vorzugsweise kann die Blisterverpackung eine Unterschale mit einer den Aufnahmeraum für das Produkt ausbildenden Vertiefung aufweisen. Eine solche Unterschale einfach mit einer zur lagestabilen Positionierung von Haltevorrichtung und Produkt ausreichenden Stabilität und Form ausgebildet sein. Durch eine an der Unterschale in bekannter Weise angeordnete Deckelfolie kann der Aufnahmeraum in der jeweils gewünschten Weise verschlossen sein, insbesondere hermetisch und/oder steril.

Nach einer weiteren Ausführungsform der Erfindung kann die Verpackung ein Trägerelement zum Beispiel nach Art eines Siebkorbs aufweisen. Dieses kann zusammen mit der Haltevorrichtung und dem daran gehaltenen Werkzeug, insbesondere mit einer Mehrzahl an Haltevorrichtungen mit dem jeweils daran gehaltenen Werkzeug, in der Verpackung angeordnet sein. Mittels eines solchen Trägerelements können mehrere Haltevorrichtung mit darin gehaltenen Produkten besonders einfach und gleichzeitig gehandhabt werden, zum Beispiel einer Sterilisierung zugeführt und/oder auf einem Instrumentierungstisch abgestellt werden.

Nach einer weiteren Ausführungsform der Erfindung kann die Haltevorrichtung kodiert sein, beispielsweise indem sie farbig ausgebildet ist. Auf diese Weise kann mit der Erfindung besonders einfach ein System bereitgestellt werden, mit dem nicht nur unterschiedliche Produkte für einen Nutzer einfach unterscheidbar kodiert sein können, sondern darüber hinaus eine solche Kodierung oder Kennzeichnung bestimmter Produkte auch nach den Entpacken des Produkts aus der Verpackung dem jeweiligen Produkt zugeordnet beibehalten werden kann. Insbesondere sind so bereits entpackte und für eine Verwendung auf einem Instrumentierungstisch oder in einer Sterilisationseinrichtung befindliche Werkzeuge für den Anwender besonders einfach und sicher zu erkennen. Eine Identifikation unterschiedlicher Produkte und/oder Haltevorrichtung kann vorzugsweise über unterschiedliche Farbgebungen der Haltevorrichtungen erfolgen, beispielsweise im Hinblick auf deren Indikation oder auf bestimmte Produktgruppen, etc. Auf diese Weise können Fehlkombinationen von Produkten durch die Farbgebung der insbesondere als Kunststoffspritzteil ausgebildeten Haltevorrichtung in und außerhalb der Verpackung vermieden werden.

Nach einer weiteren Ausführungsform der Erfindung kann die Haltevorrichtung als Kunststoffformteil ausgebildet sein. Wenn sie nach einer Fortbildung der Erfindung als Mehrkomponentenspritzguss ausgebildet ist, kann es zum Beispiel mit einer besonders rutschfesten Standfläche versehen sein. Dies ermöglicht eine stabile Anordnung der Haltevorrichtung auf einem Instrumentiertisch und/oder einem Trägerelement wie einem Siebkorb während eines Aufbereitungsprozesses. Außerdem kann so die Handhabbarkeit der Haltevorrichtung bei der Entnahme des ungebrauchten Produkts wie auch bei der Einführung eines gebrauchten Werkzeugs ungemein erleichtern werden.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 eine perspektivische Ansicht einer Ausführungsform einer Verpackung nach der Erfindung,
Fig. 2 eine perspektivische Ansicht einer Ausführungsform einer Verpackung nach der Erfindung,
Fig. 3 eine perspektivische Ansicht einer Haltevorrichtung mit darin gehaltenem Werkzeug,
Fig. 4 eine perspektivische Ansicht einer Haltevorrichtung mit darin gehaltenem Werkzeug,
Fig. 5 ein vergrößertes Detail der Fig. 4,
Fig. 6 eine Mehrzahl an Haltevorrichtungen mit jeweils einen darin gehaltenen Werkzeug,
Fig. 7 eine Schnittansicht einer Haltevorrichtung durch deren Haltestruktur quer zur Längsachse,
Fig. 8 eine Schnittansicht einer Haltevorrichtung in Richtung der Längsachse,
Fig. 9 eine Aufsicht auf die Haltevorrichtung der Figur 2 und
Fig. 10 eine Schnittansicht der Haltevorrichtung der Figur 9.

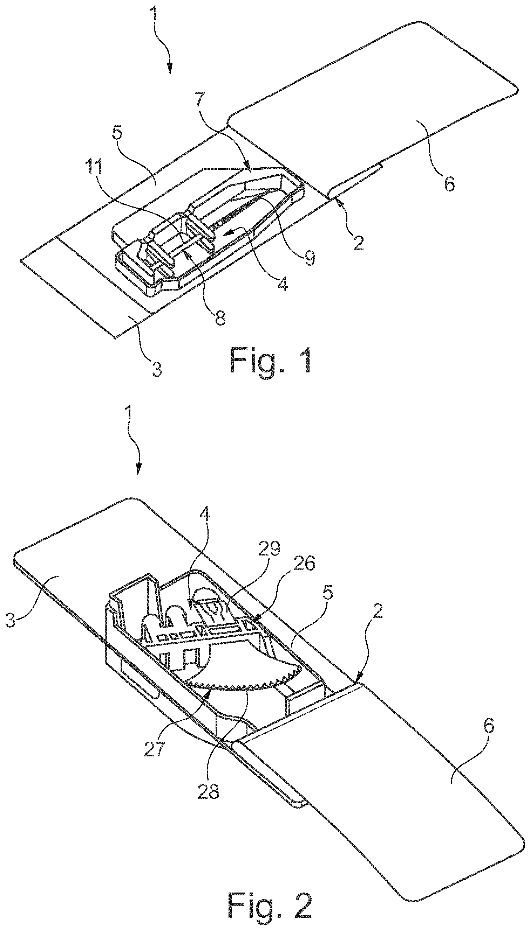

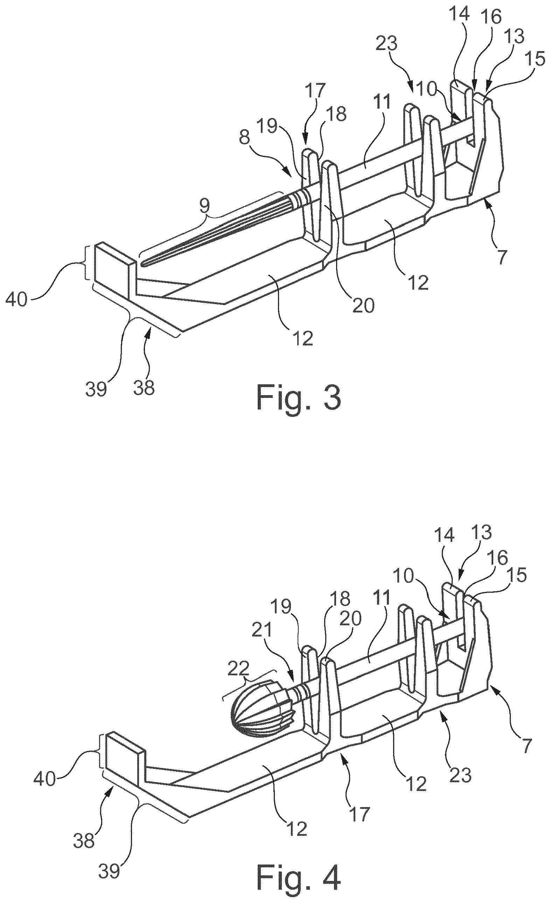

Die Figuren 1 und 2 zeigen jeweils eine Ausführungsform einer Verpackung 1 nach der Erfindung. Diese umfasst eine Umverpackung 2 in Form eines Blisters 2 mit einer Unterschale 3 mit einer darin ausgebildeten Vertiefung 4 und einem die Vertiefung 4 umgebenden Rand 5. Die Umverpackung 2 umfasst außerdem eine an dem umlaufenden Rand 5 der Unterschale 3 angeordnete und die Vertiefung 4 hermetisch dicht verschließende Deckelfolie 6, die in den Figuren 1 und 3 jeweils in einem teilweise geöffneten Zustand gezeigt ist. Sowohl die Unterschale 3 wie auch die Deckelfolie 6 bestehen in erfindungsgemäß aus einem resorbierbaren Material.

In der Vertiefung 4 der Umverpackung 2 der Figur 1 ist eine Haltevorrichtung 7 mit einem darin gehaltenen Werkzeug 8 als Beispiel für ein verpacktes Produkt angeordnet (siehe auch Figur 3). Das Werkzeug 8 ist im vorliegenden Ausführungsbeispiel ein Bohrer 8, an dessen distalen Ende ein Wirkabschnitt 9 in Form eines Bohrkopfes 9 und an dessen proximalen Ende eine Koppelstruktur 10 zum Anordnen des Werkzeugs 8 in einer nicht dargestellten Werkzeugaufnahme einer Antriebsgriffeinheit ausgebildet sind. Zwischen dem Wirkabschnitt 9 und der Koppelstruktur 10 weist das Werkzeug 8 einen langgestreckten Werkzeugschaft 11 auf.

Die Haltevorrichtung 7 der Ausführungsform der Figur 1 ist in Figur 3 ohne die Umverpackung 2 gezeigt. Sie ist als Kunststoffformteil ausgebildet, besteht vollständig aus einem resorbierbaren Material und weist eine Grundplatte 12 auf. Am proximalen Ende der Grundplatte 12 ist eine erste Haltestruktur 13 ausgebildet. Diese umfasst zwei zueinander parallele, sich von der Grundplatte 12 im Wesentlichen jeweils orthogonal in Richtung des Werkzeugs 8 erstreckende und mit der Grundplatte 12 verbundene Haltearme 14, 15. Zwischen den Haltearmen 14, 15 ist ein Schlitz 16 ausgebildet, als Aufnahme für die Koppelstruktur 10 des Werkzeugs 8. Mittig der Grundplatte 12 ist eine zweite Haltestruktur 17 ausgebildet, die sich von der Grundplatte 12 in die gleiche Richtung erstreckt wie die erste Haltestruktur 13. Die zweite Haltestruktur 17 besitzt ebenfalls zwei einander gegenüberliegende und zwischen sich einen Aufnahmeschlitz 18 ausbildende Haltearme 19, 20, die beide zur Grundplatte 12 im Wesentlichen orthogonal angeordnet sind. Zwischen der ersten Haltestruktur 13 und der zweiten Haltestruktur 17 ist eine dritte Haltestruktur 23 ausgebildet, die im Wesentlichen der zweiten Haltestruktur 17 gleicht und daher nicht weiter beschrieben wird.

Figur 4 zeigt die Haltevorrichtung 7 der Figur 3 mit einem anderen Werkzeug 21, hier in Form eines Fräsaufsatzes 21. Der Fräsaufsatz 21 weist wie der Bohrer 8 der Figuren 1 und 3 eine Koppelstruktur 10 und einen Werkzeugschaft 11 auf und unterscheidet sich vom Bohrer 8 durch seinen als Fräskopf 22 ausgebildeten distalen Wirkabschnitt 22. Dennoch können durch die Erfindung der Bohrer 8 und der Fräskopf 21 in gleichen Haltevorrichtungen 7 aufgenommen werden.

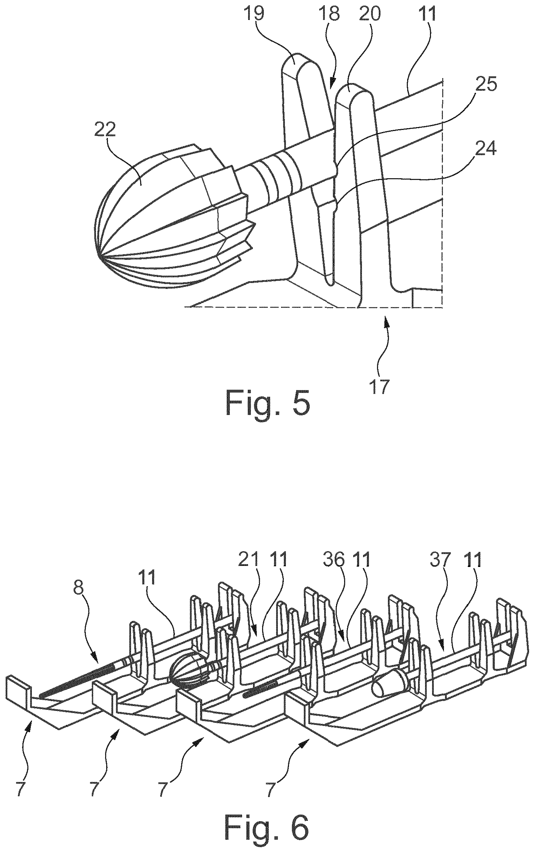

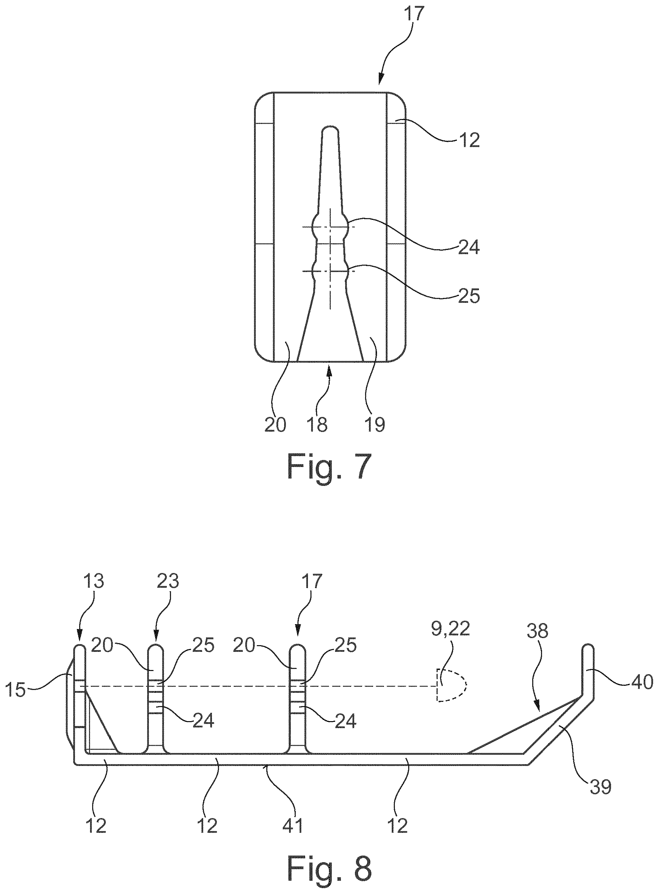

Figur 5 zeigt die zweite Haltestruktur 17 der Haltevorrichtung 7 in einer vergrößerten Detailansicht. Die einander zugewandten Seitenflächen der Haltearme 19, 20 sind mit jeweils zwei übereinander angeordneten Einbuchtungen 24, 25 von unterschiedlicher Größe versehen, jeweils zur Aufnahme von Werkzeugschäften 11 unterschiedlichen Durchmessers (siehe auch Figur 7, die die zweite Haltestruktur 17 in einer Schnittansicht quer zur Längsachse des Werkzeugs 8, 21 zeigt). Die Haltearme 19, 20 weisen gewisse elastische Federeigenschaften auf und können relativ zueinander eine Federbewegung ausführen, so dass sich der Schlitz 18 bei einem Einbringen eines Werkzeugs 8, 21 aufweiten kann und die Haltearme 19, 20 in ihre ursprüngliche Position zurückfedern, sobald der Schaft 11 des entsprechenden Werkzeugs 8, 21 in der Einbuchtung 24, 25 angeordnet ist. Auf diese Weise ist das Werkzeug 8, 21 sicher in der Haltevorrichtung 7 gehalten. Es kann insbesondere zwischen den Haltearmen 19, 20 geklemmt gehalten sein.

An dem der ersten Haltestruktur 13 gegenüberliegenden (wirkabschnittseitigen) Ende der Grundplatte 12 ist an dieser eine Schutzlasche 38 angeordnet. Die Schutzlasche 38 ragt von der Grundplatte 12 zunächst mit einem Schrägabschnitt 39 in einem schrägen Winkel (hier von etwa 45°) ab und ist dann um einen weiteren Winkel zu einem Endabschnitt 40 gebogen, so dass ihr von der Grundplatte 12 abgewandtes Ende in etwa quer zu dieser angeordnet ist. Wie insbesondere Figur 8 zeigt, ragt die Schutzlasche 38 derart weit von der Grundplatte 12 ab, dass das jeweilige distale Ende des Wirkabschnitts 9, 22 berührungsfrei abgedeckt ist. Diese Ausgestaltung der Schutzlasche 38 schafft eine gute Griffmöglichkeit in Form des Schrägabschnitts 39 zum Ergreifen durch einen Nutzer, zum Beispiel aus der in Figur 6 gezeigten Position von einem Instrumentierungstisch, wobei der Wirkabschnitt verdeckt ist, so dass dessen Kontaktierung durch den Nutzer sicher verhindert und damit ein Verletzungsrisiko minimiert werden kann. Ein weiterer Aspekt des Schrägabschnitts 39 kann sein, dass diese Schräge nach dem Prinzip des Poka Yoke im Rahmen der Fertigung / Verpackung dazu dient, die Haltevorrichtung 7 lagerichtig und richtig herum in der Umverpackung 2 ablegen zu können.

Figur 6 zeigt, dass die Haltevorrichtung 7 zur Aufnahme und zum Halten von unterschiedlichen Werkzeugen 8, 21 geeignet ist, solange diese eine im Wesentlichen ähnliche Grundform mit einem Schaft 11 und einer Koppelstruktur 10 am proximalen Ende aufweisen und sich eigentlich nur hinsichtlich ihres jeweiligen Wirkabschnitts 9, 22 unterscheiden. Beispielhaft gezeigt sind in Figur 6 der Bohraufsatz 8, der Fräsaufsatz 21, ein Schraubaufsatz 36 und ein Schleifaufsatz 37, wobei die Schaftdurchmesser der Aufsätze 36, 37 kleiner sind als die der Aufsätze 8, 21, so dass die Aufsätze 36, 37 in der Einbuchtung 24 mit kleinerem Durchmesser und die Aufsätze 8, 21 in der Einbuchtung 25 mit größerem Durchmesser angeordnet sind. Des Weiteren ergibt sich aus Figur 6 wie auch aus Figur 3 die Eignung der Haltevorrichtung 7 (gleiches gilt für die im Folgenden beschriebene Haltevorrichtung 26) neben der Lagefixierung des Werkzeugs 8, 21 im verpackten Zustand, also in dem in den Figuren 1 und 2 gezeigten Zustand, als separat von der Umverpackung 2 nutzbare Haltevorrichtung genutzt zu werden, um das Werkzeug abzustellen, beispielsweise bei einer Bereitstellung im Rahmen einer OP oder bei einer Sterilisierung. Die Unterseite 41 der Grundplatte 12, 30 bildet dazu eine Standfläche 41, auf der die Haltevorrichtung 7, 26 mit einem darin gehaltenen Werkzeug stabil abstellbar ist.

In der Vertiefung 4 der Umverpackung 2 der Figur 2 ist eine Haltevorrichtung 26 mit einem darin gehaltenen Werkzeug 27 angeordnet. Das Werkzeug 27 ist im vorliegenden Ausführungsbeispiel ein Sägeaufsatz 27, an dessen distalen Ende ein Wirkabschnitt 28 in Form eines Sägeblatts und an dessen proximalen Ende eine Koppelstruktur 29 zum Anordnen des Werkzeugs 27 in einer nicht dargestellten Werkzeugaufnahme einer Antriebsgriffeinheit ausgebildet sind.

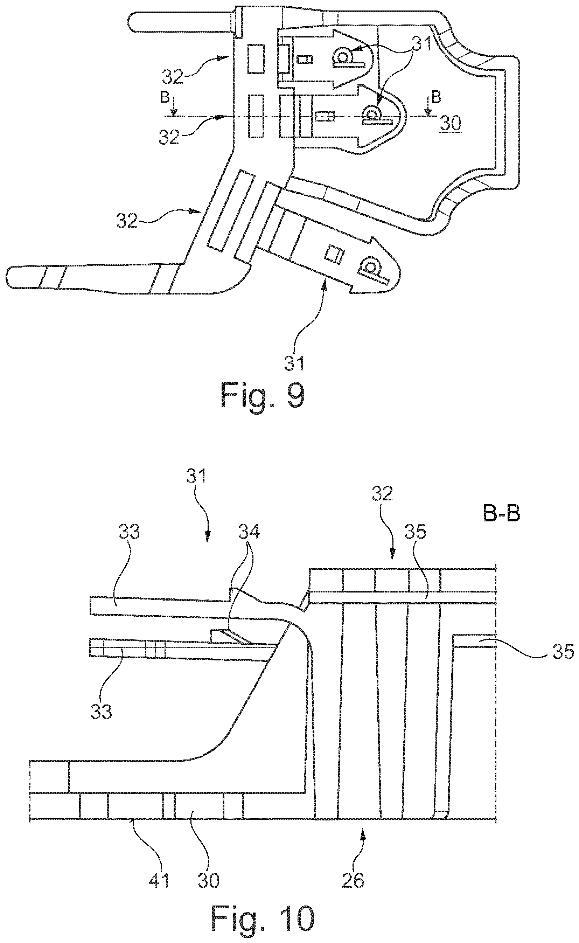

Wie die Figuren 9 und 10 zeigen, weist die Haltevorrichtung 26 eine Grundplatte 30 auf. An dieser sind eine erste Haltestruktur 31 und eine zweite Haltestruktur 32 angeordnet, die in einer Richtung quer zur Grundplatte 30 von dieser beabstandet sind. Die Haltevorrichtung 26 der Figuren 9 und 10 ist mit insgesamt drei ersten und drei zweiten Haltestrukturen 31, 32 versehen, um Sägeaufsätze unterschiedlicher Form und/oder Größe aufnehmen zu können. Die drei unterschiedlichen Haltestrukturen 31, 32 sind grundsätzlich ähnlich ausgebildet, so dass im Folgenden lediglich jeweils eine beschrieben wird. Es sei darauf hingewiesen, dass die Anzahl der drei Haltestrukturen nur beispielhaft ist, und die Haltevorrichtung 26 im Rahmen der Erfindung eine davon abweichende Anzahl von Haltestrukturen besitzen kann.

Die erste Haltestruktur 31 besitzt einen Federarm 33, der eine Federbewegung in Richtung zur Grundplatte 30 ausführen kann. Der Federarm 33 ist auf seiner von der Grundplatte 30 abgewandten Seite mit einer Raststruktur 34 hier in Form einer Rastnase 34 versehen, die mit der Koppelstruktur 29 des Sägeaufsatzes 27 zusammenwirken kann und diesen derart relativ zur zweiten Haltestruktur 32 sowie in einer Richtung parallel zur Grundplatte 30 lagefixiert. Die zweite Haltestruktur 32 ist als Schlitz 35 ausgebildet, durch den die Koppelstruktur des Sägeaufsatzes 27 hindurchgeschoben werden kann, bis diese mit der ersten Haltestruktur 31 und der Rastnase 34 verrastet. Der Schlitz 35 ist derart dimensioniert, dass der Sägeaufsatz 27 quer zur Grundplatte 30 und in der verbleibenden Richtung parallel zu dieser lagepositioniert ist. Der Schlitz 35 und der Federarm 33 sind derart ausgebildet und zueinander positioniert, dass der Sägeaufsatz 27 nur unter Einfedern des Federarms 33 in seine bestimmungsgemäße Lage gebracht werden kann, in der er mit der Rastnase 34 verrastet ist.

Zum Entnehmen des Sägeaufsatzes 27 ist der Federarm 33 durch eine Bedienperson manuell in Richtung der Grundplatte 30 zu verformen, derart, dass die Koppelstruktur des Sägeaufsatzes 27 von der Rastnase 34 gelöst werden kann und dieser in einer Richtung parallel zur Grundplatte 30 aus dem Schlitz 35 herausgezogen werden kann. Ein gebrauchter Sägeaufsatz 27 kann besonders einfach zur Zwischenlagerung und/oder Entsorgung wieder in der Haltevorrichtung 26 angeordnet werden, indem er einfach wieder in Richtung der Rastnase 34 durch den Schlitz 35 geschoben wird, wobei der Federarm 33 einfedert, bis die Rastnase 34 mit der Koppelstruktur 29 des Sägeaufsatzes 27 verrastet und dieser in der Haltevorrichtung 26 lagegesichert ist.

### Bezugszeichenliste

- 1: Verpackung
- 2: Verpackung / Umverpackung
- 3: Unterschale
- 4: Vertiefung, Aufnahmeraum
- 5: Rand
- 6: Foliendeckel
- 7: Haltevorrichtung
- 8: Produkt, Werkzeug, Bohraufsatz
- 9: Wirkabschnitt, Bohrkopf
- 10: Koppelstruktur
- 11: Werkzeugschaft
- 12: Grundplatte
- 13: erste Haltestruktur
- 14: Haltearm
- 15: Haltearm
- 16: Schlitz
- 17: zweite Haltestruktur
- 18: Aufnahmeschlitz
- 19: Haltearm, Klemmarm
- 20: Haltearm, Klemmarm
- 21: Werkzeug, Fräsaufsatz
- 22: Wirkabschnitt, Fräskopf
- 23: dritte Haltestruktur
- 24: Einbuchtung
- 25: Einbuchtung
- 26: Haltevorrichtung
- 27: Werkzeug, Sägeaufsatz
- 28: Wirkabschnitt, Sägeblatt
- 29: Koppelstruktur
- 30: Grundplatte
- 31: erste Haltestruktur
- 32: zweite Haltestruktur
- 33: Federarm
- 34: Raststruktur, Rastnase
- 35: Schlitz
- 36: Schraubaufsatz
- 37: Schleifaufsatz
- 38: Schutzlasche
- 39: Schrägabschnitt
- 40: Endabschnitt
- 41: Standfläche

## Patentansprüche

1. Verpackung (1), insbesondere Sterilverpackung (1), für ein medizintechnisches Produkt (8, 21, 27), mit einem Aufnahmeraum (4) für das medizintechnische Produkt (8, 21, 27),
**dadurch gekennzeichnet, dass**
ein dem Aufnahmeraum (4) zugewandter Abschnitt der Verpackung (1) zumindest teilweise, vorzugsweise vollständig, aus einem resorbierbaren Material besteht und die Verpackung (1) eine Umverpackung (2) und eine in dem Aufnahmeraum (4) angeordnete Haltevorrichtung (7, 26) aufweist, insbesondere eine lagebestimmt in dem Aufnahmeraum (4) angeordnete Haltevorrichtung (7, 26), in der das Produkt (8, 21, 27) gehalten ist, insbesondere lagebestimmt gehalten ist.

2. Verpackung nach Anspruch 1, **dadurch gekennzeichnet, dass** die Haltevorrichtung (7, 26) und/oder die Umverpackung (2) zumindest teilweise, vorzugsweise vollständig, aus einem resorbierbaren Material bestehen und/oder mit einer Beschichtung aus dem resorbierbaren Material versehen ist bzw. sind, zumindest in ihrem an den Aufnahmeraum (4) angrenzenden Bereich, vorzugsweise vollflächig.

3. Verpackung (1) nach einem der vorstehenden Ansprüche, **dadurch gekennzeichnet, dass** das resorbierbare Material ein Biopolymer oder ein Copolymer davon ist, oder dass das resorbierbare Material ausgewählt ist aus der Gruppe bestehend aus Gelatine, thermoplastische Stärke (TPS), Polyester, PLA (Polyactid), PLLA, PDLA oder PDLLA, PCL (Polycaprolactan) und PHB (Polyhydroxybuttersäure).

4. Verpackung (1) nach einem der Ansprüche 1 bis 3, **dadurch gekennzeichnet, dass** die Haltevorrichtung (7, 26) von der Umverpackung (2) separat ausgebildet ist und /oder die Haltevorrichtung (7, 26) ausgebildet ist, das Produkt (8, 21, 27) zumindest bereichsweise, vorzugsweise vollständig, insbesondere im Bereich eines scharfkantigen Funktions- oder Wirkabschnitts (9, 22, 28) des Produkts (8, 21, 27), von der Umverpackung (2) zu beabstanden.

5. Verpackung (1) nach einem der Ansprüche 1 bis 4, **dadurch gekennzeichnet, dass** die Haltevorrichtung (7, 26) eine Grundplatte (12, 30) aufweist, die eine Standfläche (41) zum lagestabilen Aufstellen der Haltevorrichtung (7, 26) mit einem darin gehaltenen Produkt (8, 21, 27) und/oder eine Anlagestruktur zur Anlage an der Umverpackung (2) ausbildet.

6. Verpackung (1) nach Anspruch 5, **dadurch gekennzeichnet, dass** die Haltevorrichtung (7, 26) zumindest zwei voneinander beabstandete Haltestrukturen (13, 17, 23, 31, 32) zum lagebestimmten Positionieren des Produkts (8, 21, 27) aufweist, welche Haltestrukturen (13, 17, 23, 31, 32) vorzugsweise an der Grundplatte (12, 30) angeordnet sind und sich von dieser in den Aufnahmeraum (4) hinein erstrecken, wobei sich vorzugsweise eine der Haltestrukturen (13) endseitig der Grundplatte (12, 30) und eine andere der Haltestrukturen (17, 23) im Wesentlichen mittig der Grundplatte (12, 30) angeordnet sind und/oder eine der Haltestrukturen (13, 31) zum lagebestimmten Positionieren des Produkts in einer ersten Richtung und die andere der Haltestrukturen (17, 23, 32) zum lagebestimmten Positionieren des Produkts (8, 21, 27) in einer zweiten Richtung und einer dritten Richtung ausgebildet sind, wobei die erste, zweite und dritte Richtung zueinander orthogonal sind.

7. Verpackung (1) nach Anspruch 6, **dadurch gekennzeichnet, dass** zumindest eine der Haltestrukturen (13, 31), insbesondere beide Haltestrukturen (13, 17, 23), jeweils zwei einander gegenüberliegende Haltearme (14, 15, 19, 20) aufweist bzw. aufweisen, zwischen denen ein Schlitz (16, 18) zur gegebenenfalls klemmenden Aufnahme und Halterung des Produkts (8, 21, 27) ausgebildet ist.

8. Verpackung (1) nach Anspruch 7, **dadurch gekennzeichnet, dass** sich der Schlitz (16, 18) von proximal der Grundplatte (12) in Richtung distal der Grundplatte (12) aufweitet, wobei vorzugsweise die dem Schlitz (16, 18) zugewandten Seiten der Haltearme (14, 15, 19, 20) jeweils mit zumindest einer Einbuchtung (24, 25) versehen sind, die zumindest eine Rastbucht für das in den Schlitz (16, 18) eingebrachte Produkt (8, 21, 27) bilden.

9. Verpackung (1) nach einem der Ansprüche 1 bis 8, **dadurch gekennzeichnet, dass** die Haltevorrichtung (7, 26) eine Schutzlasche (38) aufweist, die von der Grundplatte (12, 30) in den Aufnahmeraum hineinragt und ein distales Ende des Produkts (8, 21, 27) berührungslos abdeckt.

10. Verpackung (1) nach einem der Ansprüche 1 bis 9, **dadurch gekennzeichnet, dass** die Umverpackung (2) als Blister ausgebildet ist, mit einer Unterschale (3), die eine den Aufnahmeraum (4) ausbildende Vertiefung (4) aufweist, und mit einer daran angeordneten und den Aufnahmeraum verschließenden Deckelfolie (6).

11. Verpackungseinheit mit einer Verpackung (1) nach einem der vorstehenden Ansprüche und einem in dem Aufnahmeraum (4) angeordneten medizintechnischen Produkt (8, 21, 27).

## Claims

1. Package (1), in particular sterile package (1), for a medical product (8, 21, 27), comprising a receiving space (4) for the medical product (8, 21, 27),
**characterized in that**
a portion of the package (1) facing the receiving space (4) consists at least partially, preferably completely, of a resorbable material and the package (1) has an outer packaging (2) and a holding device (7, 26) arranged in the receiving space (4), in particular a holding device (7, 26) arranged in the receiving space (4) in a position-determined manner, in which the product (8, 21, 27) is held, in particular is held in a position-determined manner.

2. Package according to claim 1, **characterized in that** the holding device (7, 26) and/or the outer packaging (2) consist at least partially, preferably completely, of a resorbable material and/or is or are provided with a coating of the resorbable material, at least in their region adjacent to the receiving space (4), preferably over the entire surface.

3. Package (1) according to one of the preceding claims, **characterized in that** the resorbable material is a biopolymer or a copolymer thereof, or that the resorbable material is selected from the group consisting of gelatin, thermoplastic starch (TPS), polyester, PLA (polyactide), PLLA, PDLA or PDLLA, PCL (polycaprolactane) and PHB (polyhydroxybutyric acid).

4. Package (1) according to one of claims 1 to 3, **characterized in that** the holding device (7, 26) is designed separately from the outer packaging (2) and/or the holding device (7, 26) is designed to keep the product (8, 21, 27) at least in regions, preferably completely, in particular in the region of a sharp-edged functional or operative portion (9, 22, 28) of the product (8, 21, 27), at a distance from the outer packaging (2).

5. Package (1) according to one of claims 1 to 4, **characterized in that** the holding device (7, 26) has a base plate (12, 30) which forms a footprint (41) for placing the holding device (7, 26), with a product (8, 21, 27) held therein in a positionally stable manner, and/or which forms a contact structure for contact with the outer packaging (2).

6. Package (1) according to claim 5, **characterized in that** the holding device (7, 26) comprises at least two holding structures (13, 17, 23, 31, 32) spaced apart from each other for position-determined positioning of the product (8, 21, 27), said holding structures (13, 17, 23, 31, 32) preferably being arranged on the base plate (12, 30) and extending therefrom into the receiving space (4), wherein preferably one of the holding structures (13) is arranged at the end side of the base plate (12, 30) and another one of the holding structures (17, 23) is arranged substantially centrally with respect to the base plate (12, 30), and/or one of the holding structures (13, 31) is designed for position-determined positioning of the product in a first direction and the other one of the holding structures (17, 23, 32) is designed for position-determined positioning of the product (8, 21, 27) in a second direction and a third direction, wherein the first, second and third directions are orthogonal to each other.

7. Package (1) according to claim 6, **characterized in that** at least one of the holding structures (13, 31), in particular both holding structures (13, 17, 23), has or have in each case two holding arms (14, 15, 19, 20) which are located opposite each other and between which a slot (16, 18) is formed for receiving and holding the product (8, 21, 27) in a clamping manner, if required.

8. Package (1) according to claim 7, **characterized in that** the slot (16, 18) widens from the proximal direction of the base plate (12) towards the distal direction of the base plate (12), wherein preferably the sides of the holding arms (14, 15, 19, 20) facing the slot (16, 18) are each provided with at least one indentation (24, 25) forming at least one latching recess for the product (8, 21, 27) inserted into the slot (16, 18).

9. Package (1) according to one of claims 1 to 8, **characterized in that** the holding device (7, 26) comprises a protective lug (38) which projects from the base plate (12, 30) into the receiving space and covers a distal end of the product (8, 21, 27) without contact.

10. Package (1) according to one of claims 1 to 9, **characterized in that** the outer packaging (2) is designed as a blister, comprising a lower shell (3) which has a depression (4) forming the receiving space (4), and comprising a lid film (6) arranged thereon and closing the receiving space.

11. Packaging unit comprising a package (1) according to one of the preceding claims and a medical product (8, 21, 27) arranged in the receiving space (4).

## Revendications

1. Emballage (1), en particulier emballage stérile (1), pour un produit (8, 21, 27) médico-technique avec un espace de réception (4) pour le produit (8, 21, 27) médico-technique,
**caractérisé en ce que**
une section de l'emballage (1) tournée vers l'espace de réception (4) est constituée au moins partiellement, de préférence entièrement, d'un matériau résorbable et l'emballage (1) présente un suremballage (2) et un dispositif de maintien (7, 26) agencé dans l'espace de réception (4), en particulier un dispositif de maintien (7, 26) agencé dans une position déterminée dans l'espace de réception (4), dans lequel dispositif de maintien le produit (8, 21, 27) est maintenu, en particulier est maintenu dans une position déterminée.

2. Emballage selon la revendication 1, **caractérisé en ce que** le dispositif de maintien (7, 26) et/ou le suremballage (2) sont constitués au moins partiellement, de préférence entièrement, d'un matériau résorbable et/ou est ou sont doté(s) d'un revêtement fabriqué dans un matériau résorbable, au moins dans leur région limitrophe de l'espace de réception (4), de préférence sur toute la surface.

3. Emballage (1) selon l'une des revendications précédentes, **caractérisé en ce que** le matériau résorbable est un biopolymère ou un copolymère de celui-ci, ou **en ce que** le matériau résorbable est sélectionné dans le groupe constitué de gélatine, d'amidon thermoplastique (TPS), de polyester, de PLA (polylactide), de PLLA, PDLA ou PDLLA, de PCL (polycaprolactone) et de PHB (acide hydroxybutyrique).

4. Emballage (1) selon l'une des revendications 1 à 3, **caractérisé en ce que** le dispositif de maintien (7, 26) est conçu séparément du suremballage (2) et/ou le dispositif de maintien (7, 26) est conçu pour tenir le produit (8, 21, 27) à distance du suremballage (2), au moins par régions, de préférence entièrement, en particulier dans la région d'une section fonctionnelle ou active (9, 22, 28) à arêtes vives du produit (8, 21, 27).

5. Emballage (1) selon l'une des revendications 1 à 4, **caractérisé en ce que** le dispositif de maintien (7, 26) présente une embase (12, 30) qui forme une surface d'appui (41) pour une mise en place dans une position stable du dispositif de maintien (7, 26) avec un produit (8, 21, 27) maintenu à l'intérieur de celui-ci et/ou une structure d'installation pour une installation contre le suremballage (2).

6. Emballage (1) selon la revendication 5, **caractérisé en ce que** le dispositif de maintien (7, 26) présente au moins deux structures de maintien (13, 17, 23, 31, 32) à distance les unes des autres pour un positionnement dans une position déterminée du produit (8, 21, 27), lesquelles structures de maintien (13, 17, 23, 31, 32) sont agencées de préférence contre l'embase (12, 30) et s'étendent depuis celle-ci jusqu'à l'intérieur de l'espace de réception (4), dans lequel de préférence l'une des structures de maintien (13) est agencée sur un côté d'extrémité de l'embase (12, 30) et une autre des structures de maintien (17, 23) est agencée essentiellement au milieu de l'embase (12, 30) et/ou l'une des structures de maintien (13, 31) est conçue pour un positionnement dans une position déterminée du produit dans une première direction et l'autre des structures de maintien (17, 23, 32) est conçue pour un positionnement dans une position déterminée du produit (8, 21, 27) dans une deuxième direction et une troisième direction, dans lequel la première, deuxième et troisième directions sont perpendiculaires les unes aux autres.

7. Emballage (1) selon la revendication 6, **caractérisé en ce qu'**au moins une des structures de maintien (13, 31), en particulier les deux structures de maintien (13, 17, 23), présente ou présentent respectivement deux bras de maintien (14, 15, 19, 20) opposés l'un à l'autre, entre lesquels une fente (16, 18) est conçue pour la réception et le maintien éventuels par blocage du produit (8, 21, 27).

8. Emballage (1) selon la revendication 7, **caractérisé en ce que** la fente (16, 18) s'élargit proximalement depuis l'embase (12) et distalement dans la direction de l'embase (12), dans lequel de préférence les côtés des bras de maintien (14, 15, 19, 20) tournés vers la fente (16, 18) sont respectivement dotés d'au moins une échancrure (24, 25), qui forment au moins une anse d'encliquetage pour le produit (8, 21, 27) amené dans la fente (16, 18).

9. Emballage (1) selon l'une des revendications 1 à 8, **caractérisé en ce que** le dispositif de maintien (7, 26) présente un onglet de protection (38) qui vient en prise depuis l'embase (12, 30) jusque dans l'espace de réception et recouvre sans contact une extrémité distale du produit (8, 21, 27).

10. Emballage (1) selon l'une des revendications 1 à 9, **caractérisé en ce que** le suremballage (2) est conçu en tant qu'emballage-coque, avec une coque inférieure (3) qui présente un renfoncement (4) qui forme l'espace de réception (4), et avec une pellicule de couverture (6) agencée contre celui-ci et qui ferme l'espace de réception.

11. Unité d'emballage avec un emballage (1) selon l'une des revendications précédentes et un produit (8, 21, 27) médico-technique agencé dans l'espace de réception (4).
